# EUROPÄISCHE PATENTSCHRIFT

(11) **EP 0 546 368 B1**
(45) Veröffentlichungstag und Bekanntmachung des Hinweises auf die Patenterteilung: **04.12.1996**
(21) Anmeldenummer: 92119906.3
(22) Anmeldetag: 23.11.1992
(51) Int. Cl.: A61F 5/01

(54) **Knieorthese**
Knee brace
Orthèse du genou

(30) Priorität: 09.12.1991 DE 4140554
(43) Veröffentlichungstag der Anmeldung: 16.06.1993
(73) Patentinhaber: BIEDERMANN MOTECH GmbH, D-78054 Villingen-Schwenningen (DE)
(72) Erfinder: Lengyel, Gabor, Dipl.-Des., W-7272 Altensteig (DE)
(74) Vertreter: Prüfer, Lutz H., Dipl.-Phys.

(56) Entgegenhaltungen:
- WO-A-90/09157
- DE-A- 4 013 693
- US-A- 3 581 741
- US-A- 4 686 969
- US-A- 4 986 264

## Beschreibung

Die Erfindung betrifft eine Knieorthese nach dem Oberbegriff des Patentanspruches 1.

Eine Knieorthese mit einem Oberschenkelteil, einem Unterschenkelteil und einem die beiden Teile gelenkig verbindenden Gelenk und mit Gelenkteilen, die mit einem Verschlußgurt des Oberschenkelteiles verbundenen ersten Streben verbunden sind, ist in der DE 40 13 693 A beschrieben. Bei dieser erstreckt sich von der Außenseite des Oberschenkelgurtes über die Vorderseite diagonal eine Schlinge, die sich in 8-Form um das Bein streckt. Aus der US-PS 4.986.264 ist eine Knieorthese bekannt mit einem schalenförmigen Teil, welches auf der Vorderseite des Unterschenkels vorgesehen ist und im wesentlichen V-formig ausgebildet ist mit seinem Fußteil an dem Bereich unterhalb des Knies anliegt. Die freien Enden des schalenförmigen Teiles sind über Niete mit Streben für den Oberschenkel verbunden. Aus der EP-A-0 297 766 ist eine Knieorthese bekannt, bei der das Oberteil eine den Oberschenkel auf seiner Vorderseite umfassende Platte aufweist, die an ihrem oberen und ihrem unteren Ende mittels Verschlußgurten um den Oberschenkel legbar ist. Eine entsprechende Platte ist am Unterteil vorgesehen, die durch Verschlußgurte im oberen und unteren Bereich um den Unterschenkel anlegbar ist. Die beiden Platten sind auf ihren Außenseiten mit Verstrebungen verbunden, die mit einem inneren und einem äußeren Teil des Gelenkes verbunden sind.

Aus der US-A 4 686 969, die die Basis für den Oberbegriff des Patentanspruches 1 bildet, ist eine Knieorthese mit einem Oberschenkelteil, einem Unterschenkelteil und einem die beiden Teile gelenkig verbindenden Gelenk mit Gelenkteilen, die mit einem den Oberschenkel umfassenden Verschlußgurt des Oberschenkelteiles verbundene erste Streben und zweite Schenkel miteinander gelenkig verbinden, und mit zwei sich kreuzenden flexiblen Bändern, die sich jeweils diagonal über die Vorderseite erstrecken, bekannt.

Aufgabe der Erfindung ist es, eine Knieorthese zu schaffen, die besonders gut anpaßbar ist und als Ergebnis davon verbesserte Trageigenschaften aufweist.

Diese Aufgabe wird durch die in Patentanspruch 1 beschriebene Knieorthese gelöst.

Weiterbildungen der Erfindung sind in den Unteransprüchen gekennzeichnet.

Weitere Merkmale und Zweckmäßigkeiten der Erfindung ergeben sich aus der Beschreibung eines Ausführungsbeispieles anhand der Figuren. Von den Figuren zeigen:

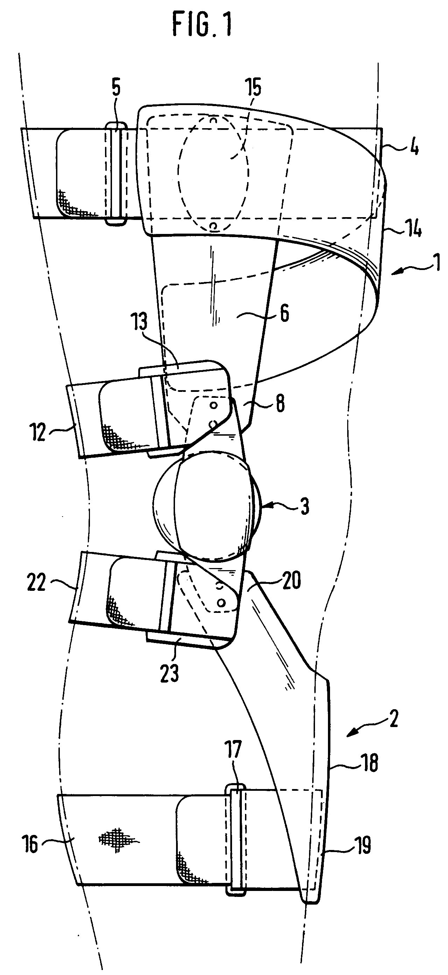

- Fig. 1: Eine Seitenansicht der Knieorthese und

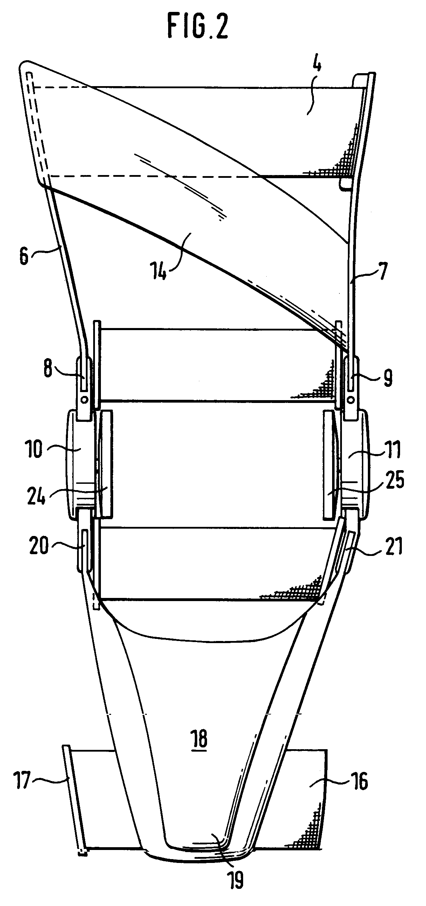

- Fig. 2: eine Vorderansicht der Knieorthese.

Die Knieorthese besteht aus dem Oberschenkelteil 1, dem Unterschenkelteil 2 und dem diese beiden Teile gelenkig miteinander verbindenden Gelenk 3.

Das Oberschenkelteil 1 weist an seinem oberen Ende einen ersten Verschlußgurt 4 auf, der als ein eine Schnalle 5 aufweisendes Band zum Umfassen des Oberschenkels ausgebildet ist. An der Außenseite und an der Innenseite des Verschlußgurtes ist dieser jeweils fest mit einer Außenstrebe 6 beziehungsweise mit einer Innenstrebe 7 verbunden. Die beiden Streben 6, 7 sind an ihrem jeweiligen unteren Ende 8, 9 mit einem Schenkel des äußeren Gelenkteiles 10 beziehungsweise inneren Gelenkteiles 11, die zusammen das Gelenk 3 bilden, verbunden. Ferner weist das Oberschenkelteil an dem unteren Abschnitt der Streben einen zweiten Verschlußgurt 12 auf. Die ser ist an einer der beiden Streben fest angebracht und weist an der anderen Strebe eine Verschlußschnalle 13 auf und ist mittels dieser Verschlußschnalle auf der Rückseite des Oberschenkels oberhalb der Kniekehle anlegbar.

Ferner ist eine Oberschenkel-Diagonalstrebe 14 vorgesehen. Diese ist mit der Innenstrebe 7 wenig oberhalb des inneren Gelenkteiles fest mit der Innenstrebe 7 verbunden. Sie erstreckt sich von diesem unteren Verbindungspunkt nach vorn und diagonal nach oben bis zu einem oberen Verbindungspunkt 15, der im wesentlichen in vertikaler Richtung gesehen oberhalb des Drehpunktes des äußeren Gelenkteiles liegt. Die Innenseite der Diagonalstrebe und die Verbindungsstelle des ersten Verschlußgurtes weisen zusammenwirkende Teile eines Klettverschlusses auf, so daß die Diagonalstrebe 14 über die Vorderseite des Oberschenkels straff anlegbar ist.

Das Unterschenkelteil weist auf seinem unteren Rand einen dritten Verschlußgurt 16 auf, der um den Unterschenkelteil herumlegbar ist und der mittels einer Schnalle 17 fest anlegbar ist. Der dritte Verschlußgurt 16 ist auf seiner Vorderseite mit einem Unterschenkelformteil 18 fest verbunden. Dieses ist als eine V-förmige Schale ausgebildet, die der üblichen Form eines Unterschenkelteiles unterhalb des Knies angepaßt ist und mit ihrem Fußteil 19 mit dem dritten Verschlußgurt verbunden ist. Die freien Enden 20, 21 sind mit den zweiten Schenkeln der beiden Gelenkteile 10 beziehungsweise 11 fest verbunden. Ferner sind die freien Enden 20, 21 mit zwei Enden eines vierten Verschlußgurtes 22 verbunden. Dieser ist an seiner einen Seite mit einem freien Ende 21 des Unterschenkelteiles 18 und mit seinem anderen freien Ende über eine Schnalle 23 mit dem anderen freien Ende des Unterschenkelteiles 18 verbunden. Mit Hilfe des dritten und vierten Verschlußgurtes und der jeweiligen Schnallen wird das Unterschenkelteil 18 gut anliegend an den Unterschenkel angelegt.

Die beiden Gelenkteile 10 und 11 weisen auf ihrer Innenseite in üblicher Weise Pelotten 24, 25 zum Anlegen an die Seiten des Knies auf.

Die Verschlußgurte sind bevorzugt aus einem einerseits eine gute Verträglichkeit garantierenden und andererseits eine ausreichende Festigkeit gewährenden Gewebematerial gebildet. Die Außenstrebe, die Innenstrebe sowie die Diagonalstrebe und das Unterschenkelteil sind aus einem thermoplastischen Kunststoff mit oder ohne Faserverstärkung wie Glasfaser, Karbonfaser oder Aramitfaser oder aus Leichtmetallen und deren Legierungen wie Titan und Aluminium gefertigt. Entscheidend ist, daß diese Materialien eine hohe Eigenstabilität und geringes Gewicht besitzen und nachformbar und korrosionsfest sind.

In dem obigen Ausführungsbeispiel ist die Oberschenkel-Diagonalstrebe 14 wenig oberhalb des inneren Gelenkteiles mit der Innenstrebe verbunden, während der obere Verbindungspunkt 15 außen liegt. Obwohl diese Ausführungsform ganz besonders bevorzugt ist, ist es gemäß einer anderen Ausführungsform auch möglich, die Oberschenkel-Diagonalstrebe zu einem oberen Verbindungspunkt zu führen, der im wesentlichen in vertikaler Richtung gesehen oberhalb des Drehpunktes des inneren Gelenkteiles liegt, während die Diagonalstrebe wenig oberhalb des äußeren Gelenkteiles fest mit der Außenstrebe verbunden ist.

## Patentansprüche

1. Knieorthese mit einem Oberschenkelteil (1), einem Unterschenkelteil (2) und einem die beiden Teile (1, 2) gelenkig verbindenden Gelenk (3) mit Gelenkteilen (10, 11), die mit einem den Oberschenkel umfassenden Verschlußgurt (4) des Oberschenkelteiles (1) verbundene erste Streben (6, 7) und zweite Schenkel miteinander gelenkig verbinden, mit einem sich von einer ersten Seite über die Vorderseite diagonal zu einem in Richtung zu dem Gelenk versetzten Ort auf der zweiten Seite diagonal erstreckenden Verbindungsteil (14), dadurch gekennzeichnet, daß das Verbindungsteil (14) als eine Verbindungsstrebe (14) aus einem Material hoher Eigenstabilität, wie thermoplastischer Kunststoff mit oder ohne Faserverstärkung wie Glasfaser, Karbonfaser oder Aramitfaser, oder Leichtmetalle und deren Legierungen wie Titan oder Aluminium, ausgebildet ist.

2. Knieorthese nach Anspruch 1,
dadurch gekennzeichnet, daß die erste Seite die Außenseite und die zweite Seite die Innenseite ist.

3. Knieorthese nach Anspruch 1,
dadurch gekennzeichnet, daß die erste Seite die Innenseite und die zweite Seite die Außenseite ist.

4. Knieorthese nach einem der Ansprüche 1 bis 3,
dadurch gekennzeichnet, daß das Unterschenkelteil (2) einen unteren Verschlußgurt (16) und ein diesen mit den zweiten Schenkeln verbindendes schalenförmiges Teil (18) aufweist.

5. Knieorthese nach einem der Ansprüche 1 bis 4,
dadurch gekennzeichnet, daß die ersten Streben (6, 7) und/oder das Unterschenkelteil (2) aus einem Material hoher Eigenstabilität, wie thermoplastischer Kunststoff mit oder ohne Faserverstärkung wie Glasfaser, Karbonfaser oder Aramitfaser, oder Leichtmetalle und deren Legierungen wie Titan oder Aluminium, gebildet sind.

## Claims

1. Knee orthesis with an above-knee part (1), a below-knee part (2), and a hinge (3) connecting the two parts (1, 2) in an articulated manner and having hinge parts (10, 11) which connect together, in an articulated manner, first braces (6, 7) connected to a closure strap (4) of the above-knee part (1) surrounding the upper leg, and second flanges; and with a connecting part (14) which extends diagonally from a first side and across the front side to a location on the second side which is offset in the direction of the hinge; characterized in that the connecting part (14) is designed as a connecting brace (14) made of a material with high inherent stability, such as a thermoplastic with or without fibre reinforcement, such as glass fibre, carbon fibre or aramid fibre, or light metals or their alloys, such as titanium or aluminium.

2. Knee orthesis according to Claim 1, characterized in that the first side is the outer side and the second side is the inner side.

3. Knee orthesis according to Claim 1, characterized in that the first side is the inner side and the second side is the outer side.

4. Knee orthesis according to one of Claims 1 to 3, characterized in that the below-knee part (2) has a lower closure strap (16) and a shell-shaped part (18) connecting this closure strap (16) to the second flanges.

5. Knee orthesis according to one of Claims 1 to 4, characterized in that the first braces (6, 7) and/or the below-knee part (2) are made of a material with high inherent stability, such as a thermoplastic with or without fibre reinforcement, such as glass fibre, carbon fibre or aramid fibre, or tight metals or their alloys, such as titanium or aluminium.

## Revendications

1. Orthèse du genou comportant un élément de cuisse (1), un élément de jambe (2) et une articulation (3) assemblant les deux éléments (1, 2) par articulation et comportant des éléments articulés (10, 11) qui assemblent par articulation les premières barres (6, 7) assemblées avec une sangle de fermeture (4) entourant la cuisse et faisant partie de l'élément de cuisse (1), avec une seconde jambe, un élément d'assemblage (14) s'étendant en diagonale d'un premier côté au second côté en passant par la face avant en diagonale par rapport à un endroit décalé vers l'articulation, caractérisée en ce que l'élément d'assemblage (14) est réalisé comme une barre d'assemblage (14) dans un matériau présentant une grande stabilité propre, tel qu'un thermoplaste avec ou sans renfort de fibres telles que des fibres de verre, des fibres de carbone ou des fibres aramides ou des métaux légers et leurs alliages tels que du titane ou de l'aluminium.

2. Orthèse du genou selon la revendication 1, caractérisée en ce que le premier côté est le côté extérieur et le second côté le côté intérieur.

3. Orthèse du genou selon la revendication 1, caractérisée en ce que le premier côté est le côté intérieur et le second côté le côté extérieur.

4. Orthèse du genou selon l'une quelconque des revendications 1 à 3, caractérisée en ce que l'élément de jambe (2) présente une sangle de fermeture (16) inférieure et un élément en forme de coque (18) assemblant cette sangle avec la seconde jambe.

5. Orthèse du genou selon l'une quelconque des revendications 1 à 4, caractérisée en ce que les premières barres (6, 7) et/ou l'élément de jambe (2) sont réalisés dans un matériau présentant une grande stabilité propre, tel qu'un thermoplaste avec ou sans renfort de fibres telles que des fibres de verre, des fibres de carbone ou des fibres aramides ou des métaux légers et leurs alliages tels que du titane ou de l'aluminium.
